# EUROPEAN PATENT APPLICATION

(11) **EP 3 254 605 A1**
(43) Date of publication of application: **13.12.2017**
(21) Application number: 16891591.6
(22) Date of filing: 13.10.2016
(51) Int. Cl.: A61B 1/00, G02B 23/24

(54) **CONDUIT-SWITCHING PISTON AND ENDOSCOPE**

(30) Priority: 24.02.2016 JP 2016033400
(71) Applicant: Olympus Corporation, Hachioji-shi, Tokyo 192-8507 (JP)
(72) Inventor: SUZUKI Mizuki, Hachioji-shi, Tokyo 192-8507 (JP); MATSUURA Nobuyuki, Tokyo 192-8507 (JP); UENO Haruhiko, Tokyo 192-8507 (JP); URUSHIDO Masaharu, Tokyo 192-8507 (JP)
(74) Representative: Schicker, Silvia
(86) International application number: PCT/JP2016/080314
(87) International publication number: WO 2017/145435

(57) **Abstract**

A conduit switching piston 10 is provided that is fitted and inserted in an advanceable and retractable manner into a cylinder to which a plurality of conduits are connected, and is configured to switch communication states of the plurality of conduits. The conduit switching piston 10 includes: a shaft member 45; a flat portion that is formed along an extending direction E on an outer circumferential face 45g of the shaft member 45, and that has two edge portions facing each other on the outer circumferential face 45g and extending along the extending direction; a plurality of seal portions 53, 54, 55 and 56 formed having set intervals E1, E2 and E3 with respect to each other along the extending direction E on the outer circumferential face 45g of the shaft member 45, and configured to elastically contact an inner circumferential face of the cylinder; and a connecting portion 59 formed integrally with the seal portions 53, 54, 55 and 56 on the flat portion, and configured to connect the seal portions 53 and 54, the seal portions 54 and 55, and the seal portions 55 and 56 that are respectively adjacent along the extending direction E.

## Description

### Technical Field

The present invention relates to a conduit switching piston that is fitted and inserted in an advanceable and retractable manner into a cylinder to which a plurality of conduits are connected, and is configured to switch communication states of the plurality of conduits, and also relates to an endoscope.

### Background Art

By inserting an elongated insertion portion of an endoscope into a subject, the endoscope can observe an inside of the subject using an observation lens provided at a distal end of the insertion portion.

Further, a configuration is known in which an air/water feeding nozzle is provided at the distal end of the insertion portion. The air/water feeding nozzle is a component that removes dirt on an observation lens by supplying a fluid to the observation lens and, for example, in the case of an endoscope for medical use, supplies a gas into a body cavity to expand the inside of the body cavity and secure an observation field of view within the body cavity.

The air/water feeding nozzle is connected to a distal end of an air/water feeding conduit provided inside the insertion portion. The air/water feeding conduit, for example, is branched into an air feeding conduit and a water feeding conduit inside the insertion portion. The air feeding conduit is connected to a gas supply source and a liquid supply source, respectively. The water feeding conduit is connected to a liquid supply source.

A configuration is also known in which a conduit switching piston is provided in an operation portion that is connected to the proximal end of the insertion portion of an endoscope.

The conduit switching piston is configured to switch a fluid to be supplied from the air/water feeding nozzle between a gas and a liquid, and also switches between a state in which fluid is supplied from the air/water feeding nozzle and a state in which the supply of fluid from the air/water feeding nozzle is cut off.

Inside the operation portion, the conduit switching piston is fitted and inserted in an advanceable and retractable manner into a cylinder that is connected at a position that is partway along the air feeding conduit and the water feeding conduit.

An upstream side and a downstream side of the air feeding conduit are each connected to the cylinder, and an upstream side and a downstream side of the water feeding conduit are each connected to the cylinder.

The conduit switching piston is configured to switch a communication state between the upstream side and downstream side of the air feeding conduit between a state in which communication is allowed and a state in which communication is cut off, and also switch the communication state between the upstream side and downstream side of the water feeding conduit between a state in which communication is allowed and a state in which communication is cut off.

Specifically, the conduit switching piston has a configuration that includes a shaft member, and a plurality of seal portions that are covered over the outer circumferential face of the shaft member and are configured to butt against the inner circumferential face of the cylinder in a watertight and airtight manner.

As is known, the conduit switching piston changes the contact positions of the plurality of seal portions with respect to the inner circumferential face of the cylinder by causing the shaft member to advance or retract with respect to the cylinder.

Thus, the conduit switching piston has a configuration that switches the communication state between the upstream side and downstream side of the air feeding conduit between a state in which communication is allowed and a state in which communication is cut off, and also switches the communication states between the upstream side and downstream side of the water feeding conduit between a state in which communication is allowed and a state in which communication is cut off.

More specifically, a through-hole is formed in the radial direction in the shaft member. Further, in the shaft member, a communication passage that communicates with the through-hole and also communicates with a leak hole provided in an upper portion of the shaft member is formed along the extending direction of the shaft member.

In a fluid cut-off state in which communication between the air feeding conduit and the water feeding conduit is cut off by the plurality of seal portions, because gas that is supplied to the cylinder from the upstream side of the air feeding conduit is released to the atmosphere via the through-hole, the communication passage and the leak hole, the supply of fluid from the air/water feeding nozzle is cut off.

In a leak-hole blockage state which is entered at a time that the leak hole is blocked by an operator when in the fluid cut-off state, a seal portion that blocks communication between the upstream side and downstream side of the air feeding conduit inside the cylinder is deformed by gas supplied into the cylinder from the upstream side of the air feeding conduit.

As a result, because the seal portion that blocks communication between the upstream side and downstream side of the air feeding conduit does not contact against the inner circumferential face of the cylinder, the upstream side and downstream side of the air feeding conduit communicate, and hence gas is discharged from the air/water feeding nozzle.

In a state in which the shaft member was moved inside the cylinder from the leak-hole blockage state, the contact positions of the plurality of seal portions with respect to the inner circumferential face of the cylinder change.

Consequently, as a result of the seal portion that blocks communication between the upstream side and downstream side of the water feeding conduit inside the cylinder separating from the cylinder inner circumferential face, the upstream side and downstream side of the water feeding conduit communicate with each other.

In addition, inside the cylinder, communication between the upstream side and downstream side of the air feeding conduit is cut off by a seal portion that blocks communication between the upstream side and the downstream side of the air feeding conduit.

As a result, because gas is supplied to the liquid supply source, a liquid that is pushed out from the liquid supply source by the gas is discharged from the air/water feeding nozzle through the water feeding conduit that is caused to communicate therewith.

In this case, generally a seal portion is used that has a configuration in which an O-shaped ring or the like is covered over the outer circumferential face of a shaft member made of stainless steel or the like.

However, because the O-shaped ring slidingly moves over the inner circumferential face of the cylinder accompanying movement of the shaft member, the position of the O-shaped ring is liable to be displaced with respect to the outer circumferential face of the shaft member.

Hence, a configuration is known in which a mounting-strength reinforcement portion for an O-shaped ring is provided on an outer circumferential face of a shaft member.

However, according to such a configuration, there is the problem that the number of components increases and the configuration of the conduit switching piston becomes complex.

In view of this problem, in U.S. Patent No. 9,161,680 a configuration of a conduit switching piston is disclosed in which a shaft member is injection molded from resin or the like, a groove portion is formed along the shaft member in the outer circumferential face of the shaft member, and a sealing unit made of resin or the like is injection molded using a mold in the groove portion.

Note that the sealing unit includes a plurality of seal portions and a connecting portion configured to connect the seal portions along the extending direction thereof.

Because the sealing unit is formed integrally with the shaft member by being injection molded in the groove portion of the shaft member, the fixing strength with respect to the outer circumferential face of the shaft member is improved without using a strength reinforcement portion.

However, in the conduit switching piston disclosed in U.S. Patent No. 9,161,680, the groove portion in which the sealing unit is injection molded along the extending direction of the shaft member is formed in the outer circumferential face of the shaft member.

Therefore, there is the problem that the shape of the mold to be used when performing injection molding of the sealing unit is complex, and thus the manufacturing cost increases.

The present invention has been made in view of the above described problems, and an object of the present invention is to provide a conduit switching piston having a configuration in which a plurality of seal portions are integrally formed with respect to the outer circumferential face of a shaft member inexpensively and in a manner that improves the fixing strength, and to also provide an endoscope.

### Disclosure of Invention

### Means for Solving the Problem

A conduit switching piston according to one aspect of the present invention is a conduit switching piston that is fitted and inserted in an advanceable and retractable manner into a cylinder to which a plurality of conduits are connected, the conduit switching piston being configured to switch communication states of the plurality of conduits, the conduit switching piston including: a shaft member; a flat portion formed along an extending direction of the shaft member on an outer circumferential face of the shaft member, and having two edge portions facing each other on the outer circumferential face and extending along the extending direction; a plurality of seal portions formed having a set interval with respect to each other along the extending direction on the outer circumferential face of the shaft member, and configured to elastically contact an inner circumferential face of the cylinder; and a connecting portion formed on the flat portion integrally with the seal portions, and configured to connect the seal portions which are adjacent along the extending direction.

An endoscope according to one aspect of the present invention is equipped with the conduit switching piston according to claim 1.

### Brief Description of the Drawings

Fig. 1 is a view that schematically illustrates an endoscope apparatus including an endoscope in which a conduit switching piston of the present embodiment is provided;
Fig. 2 is a view that schematically illustrates a conduit configuration that communicates with an air/water feeding nozzle in the endoscope in Fig. 1, together with an air/water feeding switching apparatus and a water feeding tank;
Fig. 3 is a partial cross-sectional view that schematically illustrates the configuration of a conduit switching apparatus shown in Fig. 2;
Fig. 4 is a partial cross-sectional view that schematically illustrates a state in which a leak hole of an air/water feeding button shown in Fig. 3 is blocked, and a downstream-side air feeding conduit and an upstream-side air feeding conduit inside a cylinder are caused to communicate;
Fig. 5 is a partial cross-sectional view that schematically illustrates a state in which an air/water feeding button shown in Fig. 3 is pushed down to thereby cut off communication between the downstream-side air feeding conduit and the upstream-side air feeding conduit inside the cylinder and to also cause a downstream-side water feeding conduit and an upstream-side water feeding conduit to communicate;
Fig. 6 is a perspective view illustrating a piston main body and a sealing unit in the conduit switching piston shown in Fig. 3;
Fig. 7 is a perspective view in which, relative to Fig. 6, the sealing unit is removed and only the piston main body is illustrated;
Fig. 8 is a top view of the conduit switching piston in Fig. 6 as seen from a VIII direction in Fig. 6;
Fig. 9 is a side view of the conduit switching piston in Fig. 6 as seen from an IX direction in Fig. 6;
Fig. 10 is a view illustrating the conduit switching piston along an X-X line in Fig. 8, and which illustrates a cross-section of only one half of the conduit switching piston;
Fig. 11 is a cross-sectional view of the conduit switching piston along an XI-XI line in Fig. 10;
Fig. 12 is a cross-sectional view of the conduit switching piston along an XII-XII line in Fig. 10;
Fig. 13 is a cross-sectional view of the conduit switching piston along an XIII-XIII line in Fig. 10;
Fig. 14 is a cross-sectional view of the conduit switching piston along an XIV-XIV line in Fig. 10;
Fig. 15 is a top view of the piston main body in Fig. 7 as seen from an XV direction in Fig. 7;
Fig. 16 is a side view of the piston main body in Fig. 7 as seen from an XVI direction in Fig. 7;
Fig. 17 is a view illustrating the piston main body along an XVII-XVII line in Fig. 15, and which illustrates a cross-section of only one half of the piston main body;
Fig. 18 is a cross-sectional view of the piston main body along an XVIII-XVIII line in Fig. 17;
Fig. 19 is a cross-sectional view of the piston main body along an XIX-XIX line in Fig. 17;
Fig. 20 is a view illustrating a modification of the conduit switching piston in which the shape of a through-hole formed in the piston main body in Fig. 10 is made elliptical, and which illustrates a cross-section of only one half of the conduit switching piston; and
Fig. 21 is an exploded perspective view of a piston main body on which a sealing unit of the conduit switching piston shown in Fig. 3 is formed, an enclosing member, an urging spring and an air/water feeding button.

### Best Mode for Carrying Out the Invention

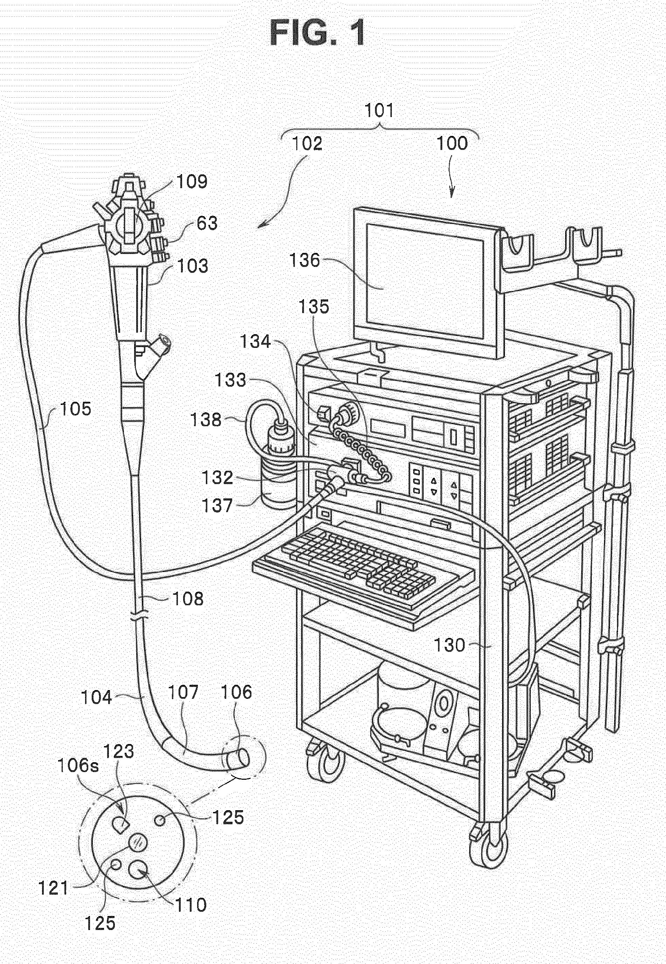

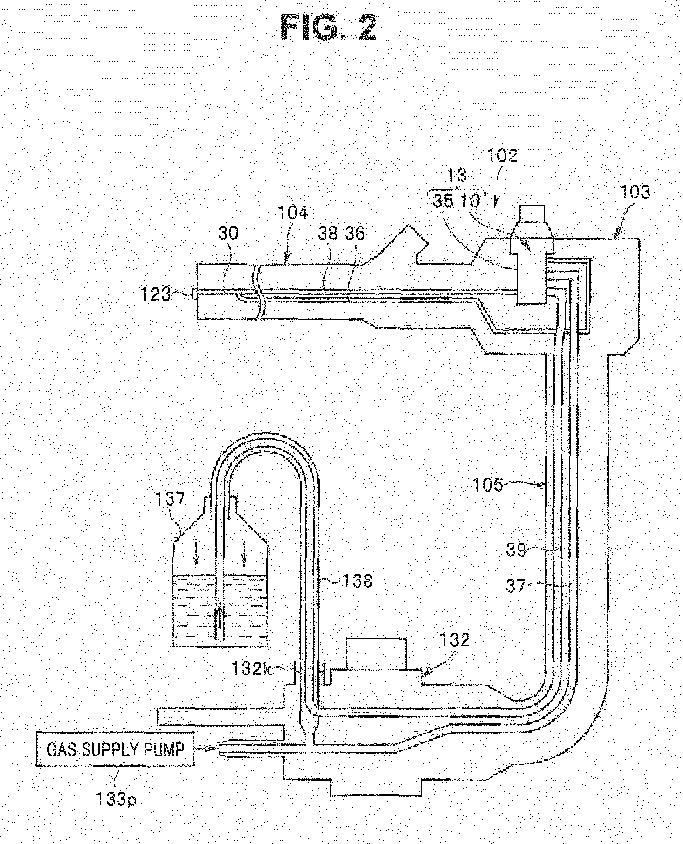

Embodiments of the present invention are described hereunder with reference to the accompanying drawings. It should be noted that the drawings are schematic ones in which the relationship between the thickness and width of each member, the thickness ratios of the members and the like are different from those of actual members. Naturally, the drawings include portions in which the dimensional relationships and ratios are different from one another. Fig. 1 is a view that schematically illustrates an endoscope apparatus including an endoscope in which a conduit switching piston of the present embodiment is provided, and Fig. 2 is a view that schematically illustrates a conduit configuration that communicates with an air/water feeding nozzle in the endoscope in Fig. 1, together with an air/water feeding switching apparatus and a water feeding tank.

As illustrated in Fig. 1, a principal part of an endoscope apparatus 101 is configured by including an endoscope 102 and peripheral apparatuses 100.

The peripheral apparatuses 100 include a light source apparatus 133, a video processor 134, a monitor 136 and a water feeding tank 137 which are placed on a rack 130.

A gas supply pump 133P (see Fig. 2) is provided inside the light source apparatus 133.

A principal part of the endoscope 102 is configured by including an insertion portion 104 configured to be inserted into a subject, an operation portion 103 that is connected to a proximal end of the insertion portion 104, a universal cord 105 that is extended from the operation portion 103, and a connector 132 provided at an extending end of the universal cord 105. The connector 132 is detachably attachable to the light source apparatus 133.

The connector 132 and the video processor 134 are electrically connected by a connection cable 135.

A tube 138 that is extended from the water feeding tank 137 is inserted through the inside of the connector 132 through a pipe sleeve 132k (see Fig. 2) of the connector 132.

The insertion portion 104 includes a distal end portion 106 that is located on a distal end side of the insertion portion 104, a bending portion 107 that is operated to bend in, for example, the four directions of upward, downward, left and right by a bending operation knob 109 provided in the operation portion 103, and a flexible tube portion 108 that is connected to a proximal end of the bending portion 107.

An opening 110 of an unshown treatment instrument insertion channel that is provided inside the endoscope 102, an observation lens 121, an air/water feeding nozzle 123 and an illuminating window 125 and the like are provided in a distal end face 106s of the distal end portion 106.

The air/water feeding nozzle 123 is configured to remove dirt adhering to the observation lens 121 by supplying a liquid toward the observation lens 121 upon an air/water feeding button 63 provided in the operation portion 3 being operated.

In addition, the air/water feeding nozzle 123 is configured to supply a gas into a subject to expand the inside of the subject to secure the observation field of view of the observation lens 121.

The illuminating window 125 is configured to supply an illuminating light into the subject. Note that, instead of the illuminating window 125, a light emitting device such as an LED may be provided in the distal end face 106s.

As illustrated in Fig. 2, a distal end of an air/water feeding conduit 30 is connected to the air/water feeding nozzle 123.

Inside the insertion portion 104, the air/water feeding conduit 30 is branched into a downstream-side air feeding conduit (hereunder, referred to simply as "air feeding conduit") 36 and a downstream-side water feeding conduit (hereunder, referred to simply as "water feeding conduit") 38.

Further, the proximal ends of the respective conduits 36 and 38 are connected to a cylinder 35 in the conduit switching apparatus 13 provided in the operation portion 3.

A distal end of an upstream-side air feeding conduit (hereunder, referred to simply as "air feeding conduit") 37 and a distal end of a downstream-side water feeding conduit (hereunder, referred to simply as "water feeding conduit") 39 are connected to the cylinder 35.

When the connector 132 is mounted to the light source apparatus 133, a proximal end of the air feeding conduit 37 is connected to the gas supply pump 133p provided inside the light source apparatus 133.

Inside the connector 132, the tube 138 that is extended from the water feeding tank 137 is connected to the air feeding conduit 37. That is, the air feeding conduit 37 is connected to the water feeding tank 137 through the tube 138.

The proximal end of the water feeding conduit 39 is positioned inside the water feeding tank 137 by inserting the proximal end side of the water feeding conduit 39 through the tube 138.

A principal portion of the conduit switching apparatus 13 is configured by including the cylinder 35, and a conduit switching piston 10 that is fitted and inserted in an advanceable and retractable manner into the cylinder 35.

Further, the conduit switching apparatus 13 is configured to switch a communication state between the air feeding conduit 37 and the air feeding conduit 36 between a state in which communication is allowed and a state in which communication is cut-off, and is configured to switch a communication state between the water feeding conduit 38 and the water feeding conduit 39 between a state in which communication is allowed and a state in which communication is cut-off.

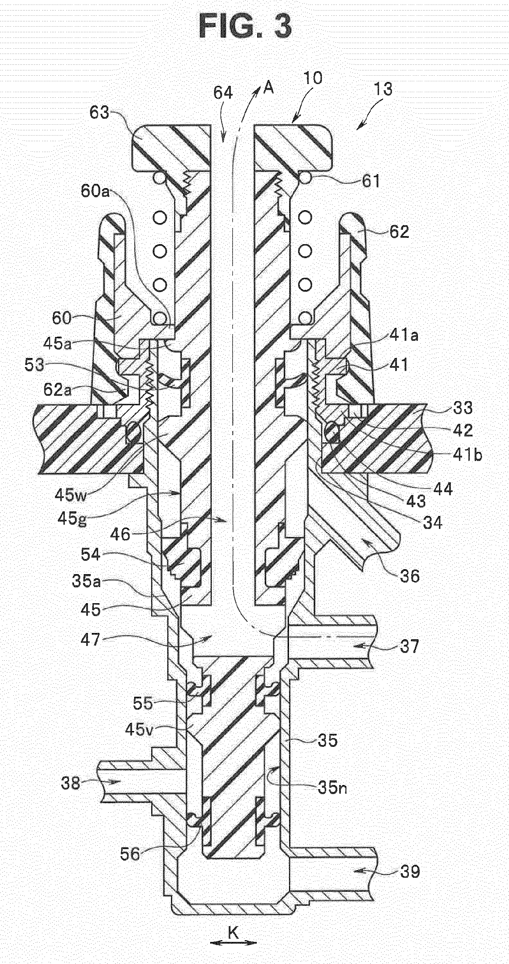

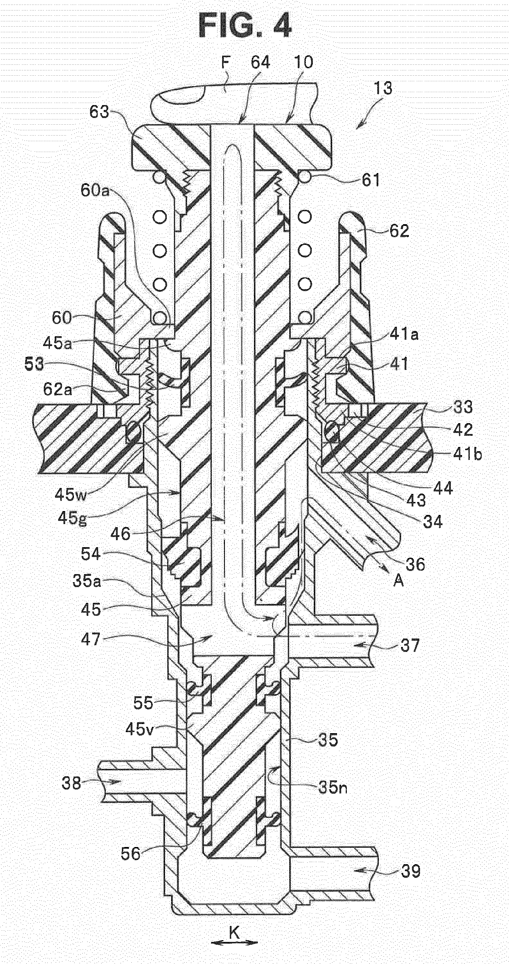

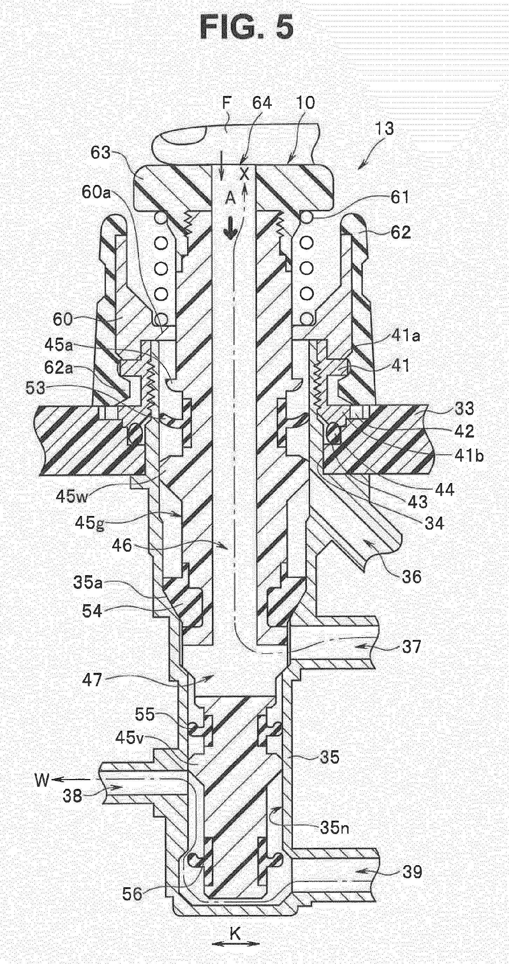

Next, the configuration of the conduit switching apparatus 13 illustrated in Fig. 2 will be described using Fig. 3 to Fig. 5. Fig. 3 is a partial cross-sectional view that schematically illustrates the configuration of the conduit switching apparatus illustrated in Fig. 2. Fig. 4 is a partial cross-sectional view that schematically illustrates a state in which a leak hole in an air/water feeding button shown in Fig. 3 is blocked to thereby cause a downstream-side air feeding conduit and an upstream-side air feeding conduit in the cylinder to communicate. Fig. 5 is a partial cross-sectional view that schematically illustrates a state in which an air/water feeding button shown in Fig. 3 is pushed down to thereby cut off communication between the downstream-side air feeding conduit and the upstream-side air feeding conduit in the cylinder, and cause a downstream-side water feeding conduit and an upstream-side water feeding conduit to communicate.

As illustrated in Fig. 3 to Fig. 5, a mounting hole 34 is formed in an outer sheathing member 33 configuring the operation portion 103 so as to penetrate through the outer sheathing member 33 in an extending direction E, described later, and the cylinder 35 is fixed in the mounting hole 34.

The cylinder 35 is formed in a substantially cylindrical shape having steps which, for example, is made from metal. In a side wall of the cylinder 35, the aforementioned air feeding conduit 37, water feeding conduit 39, air feeding conduit 36 and water feeding conduit 38 are connected so as to communicate inside the cylinder 35 in the order of the air feeding conduit 36, the air feeding conduit 37, the water feeding conduit 38 and the water feeding conduit 39 from the upper side in Fig. 3 toward the lower side.

A threaded portion is formed in the outer circumferential face of an opening portion of the cylinder 35. The cylinder 35 is fixed to the outer sheathing member 33 so as to sandwich the outer sheathing member 33 from the inner and outer sides by screwing a pipe sleeve 41 into the threaded portion of the cylinder 35 from the outer side of the outer sheathing member 33.

Note that a jig hole 42 into which a jig is inserted for rotating the pipe sleeve 41 when mounting the pipe sleeve 41 to the threaded portion of the cylinder 35 is provided in the outer circumference of the pipe sleeve 41.

The pipe sleeve 41 has an upper flange 41a and a lower flange 41b. The pipe sleeve 41 is fixed to the cylinder 35 in which the mounting hole 34 is sealed by an O-shaped ring 44 inside an annular groove 43 formed in the inner circumferential face of the mounting hole 34 being compressed by the lower flange 41b. By this means, entry of a gas or a liquid into the operation portion 103 is prevented.

The conduit switching piston 10, for example, includes a piston main body 45 that is a shaft member made from a resin. The piston main body 45 is formed, for example, by injection molding.

Inside the piston main body 45, a communication passage 46 is formed along the extending direction E of the piston main body 45.

Further, at the lower end in the extending direction E of the communication passage 46 of the piston main body 45 (hereunder, referred to simply as "lower end"), a through-hole 47 is formed that communicates with the communication passage 46 and also penetrates through the piston main body 45 in a radial direction K of the piston main body 45.

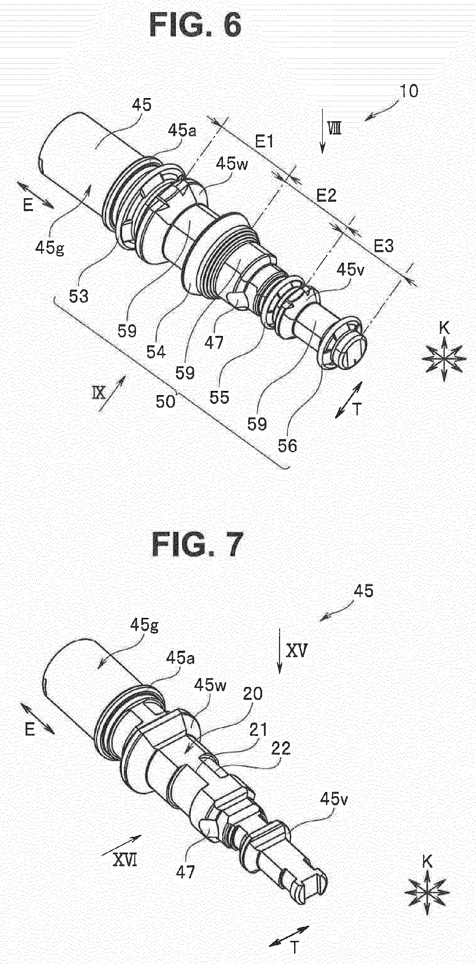

On an outer circumferential face 45g of the piston main body 45, a ring-shaped seal portion 54 of a sealing unit 50 (see Fig. 6) is formed integrally with the piston main body 45 by injection molding at a position in the vicinity of an upper portion in the extending direction E of the through-hole 47 (hereunder, referred to simply as "upper portion"). The seal portion 54 is made, for example, from a resin and is configured to elastically contact an inner circumferential face 35n of the cylinder 35.

Further, on the outer circumferential face 45g of the piston main body 45, a slider 45w having a face that butts against the inner circumferential face 35n is formed integrally with the piston main body 45 at a position that is upward in the extending direction E relative to the seal portion 54 (hereunder, referred to simply as "upward").

Note that the slider 45w is a member configured to prevent the piston main body 45 from becoming misaligned with respect to the cylinder 35 by butting against the inner circumferential face 35n.

In addition, on the outer circumferential face 45g, a ring-shaped seal portion 53 of the sealing unit 50 (see Fig. 6) that, for example, is made from a resin and is configured to elastically contact the inner circumferential face 35n of the cylinder 35 is formed integrally with the piston main body 45 by injection molding at an upper portion of the slider 45w. That is, the seal portion 53 is disposed at a position that is separated by a set interval E1 (see Fig. 6) in the upward direction from the seal portion 54 in the extending direction E.

On the outer circumferential face 45g of the piston main body 45, a ring-shaped seal portion 55 of the sealing unit 50 (see Fig. 6) is formed integrally with the piston main body 45 by injection molding at a position in the vicinity of a lower portion in the extending direction E of the through-hole 47 (hereunder, referred to simply as "lower portion").

The seal portion 55, for example, is made from a resin and is configured to elastically contact the inner circumferential face 35n of the cylinder 35. That is, the seal portion 55 is disposed at a position that is separated in a downward direction in the extending direction E (hereunder, referred to simply as "downward") by a set interval E2 (see Fig. 6) from the seal portion 54 in the extending direction E.

Further, on the outer circumferential face 45g of the piston main body 45, a slider 45v having a face that butts against the inner circumferential face 35n is formed integrally with the piston main body 45 in the vicinity of the lower portion of the seal portion 55.

Note that the slider 45v is a member configured to prevent the piston main body 45 from becoming misaligned with respect to the cylinder 35 by butting against the inner circumferential face 35n.

In addition, on the outer circumferential face 45g of the piston main body 45, a ring-shaped seal portion 56 of the sealing unit 50 (see Fig. 6) is formed integrally with the piston main body 45 by injection molding at a position that is downward relative to the slider 45v.

The seal portion 56 is made, for example, from a resin and is configured to elastically contact the inner circumferential face 35n of the cylinder 35. That is, the seal portion 56 is disposed at a position that is separated by a set interval E3 in the downward direction from the seal portion 55 (see Fig. 6) in the extending direction E.

Further, a cylindrical piston stopper 60 that is made, for example, from a rigid member is provided at the outer circumference on the upper portion side of the piston main body 45.

The piston stopper 60 has an inward flange portion 60a, and an outward flange portion 45a provided at an upper portion relative to the seal portion 53 on the outer circumferential face 45g of the piston main body 45 freely butts against the inward flange portion 60a.

An urging spring 61 configured by a coil spring is interposed between a top face in the extending direction E of the inward flange portion 60a (hereunder, referred to simply as "top face") and a bottom face in the extending direction E of the air/water feeding button 63 (hereunder, referred to simply as "bottom face") that is screwingly attached to a top end in the extending direction E of the piston main body 45 (hereunder, referred to simply as "top end").

The urging force of the urging spring 61 urges the air/water feeding button 63 upward and urges the piston stopper 60 downward.

As illustrated in Fig. 3, in a natural state, the top face of the outward flange portion 45a is butted against the bottom face of the inward flange portion 60a, and as a result the piston main body 45 is locked.

In addition, at the outer circumference of the piston stopper 60, an enclosing member 62 that is made, for example, from rubber, is provided integrally with the piston stopper 60.

An inward protrusion portion 62a provided at the lower end of the enclosing member 62 is engaged with the bottom face of the upper flange 41a of the pipe sleeve 41. Further, a leak hole 64 that communicates with the communication passage 46 is formed at a center portion of the air/water feeding button 63.

Next, an operation for switching the communication state of the air feeding conduit and an operation for switching the communication state of the water feeding conduit using the conduit switching apparatus 13 configured as described above will be briefly described.

First, as illustrated in Fig. 3, in a natural state the piston main body 45 is being pushed upward in the extending direction E by the urging force of the urging spring 61. At this time, an upward position of the piston main body 45 is defined by the top face of the outward flange portion 45a being butted against the bottom face of the inward flange portion 60a.

Further, communication between the water feeding conduit 39 and the water feeding conduit 38 inside the cylinder 35 is cut off by the seal portion 56.

In addition, communication between the air feeding conduit 37 and the air feeding conduit 36 inside the cylinder 35 is cut off by the seal portions 54 and 55.

Therefore, a gas that is supplied into the cylinder 35 through the air feeding conduit 37 from the gas supply pump 133P flows into the through-hole 47 and passes through the communication passage 46 and flows out into the atmosphere from the leak hole 64.

Next, as illustrated in Fig. 4, when the leak hole 64 is blocked by a finger F of an operator, the seal portion 54 is bent upward by the gas that is supplied into the cylinder 35, and the seal portion 54 separates from the inner circumferential face 35n of the cylinder 35.

As a result, because the air feeding conduit 37 and the air feeding conduit 36 communicate, the gas flows into the air feeding conduit 36 through the cylinder 35 from the air feeding conduit 37, and thereafter the gas flows into the air/water feeding conduit 30 from the air feeding conduit 36 and is discharged from the air/water feeding nozzle 123.

Note that, in the state illustrated in Fig. 4, communication between the water feeding conduit 39 and the water feeding conduit 38 inside the cylinder 35 remains cut off by the seal portion 56.

Next, as illustrated in Fig. 5, when an operation is performed to push the air/water feeding button 63 downward in a state in which the leak hole 64 is blocked by the finger F of the operator, inside the cylinder 35 the piston main body 45 is moved downward in the extending direction E against the urging force of the urging spring 61, and the outward flange portion 45a separates from the inward flange portion 60a in the downward direction.

As a result, because the seal portion 54 is pressed against a tapered face 35a formed in the inner circumferential face 35n of the cylinder 35 and elastically deforms and is flattened, communication between the air feeding conduit 37 and the air feeding conduit 36 inside the cylinder 35 is cut off.

In addition, accompanying downward movement of the piston main body 45, the seal portion 56 separates from the inner circumferential face 35n of the cylinder 35. As a result, the water feeding conduit 39 and the water feeding conduit 38 communicate with each other.

Hence, because the leak hole 64 is blocked and communication between the air feeding conduit 37 and the air feeding conduit 36 is cut off by the seal portion 54, the gas supplied from the gas supply pump 133P is supplied into the water feeding tank 137 through the tube 138 as illustrated in Fig. 2.

Consequently, the liquid inside the water feeding tank 137 is pushed out, and liquid that flowed into the cylinder 35 from the water feeding conduit 39 flows into the water feeding conduit 38 and furthermore flows into the air/water feeding conduit 30 and is thereafter discharged from the air/water feeding nozzle 123.

Next, the specific configuration of the conduit switching piston 10 illustrated in Fig. 2 to Fig. 5 is described using Fig. 6 to Fig. 19.

Fig. 6 is a perspective view illustrating a piston main body and a sealing unit in the conduit switching piston illustrated in Fig. 3, and Fig. 7 is a perspective view in which, relative to Fig. 6, the sealing unit is removed and only the piston main body is illustrated;

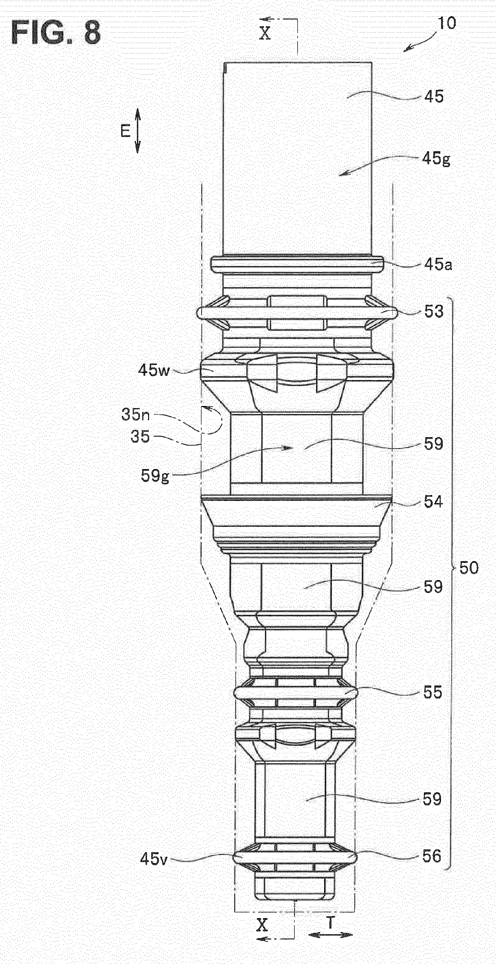

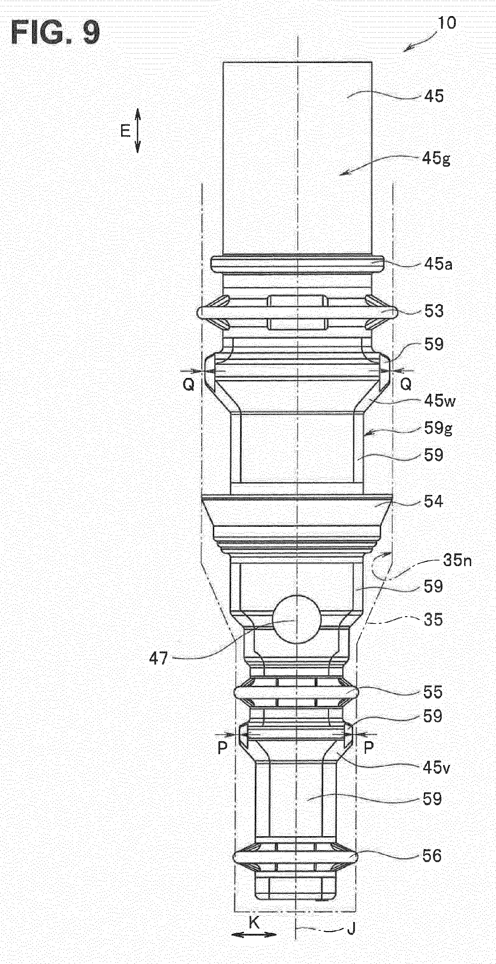

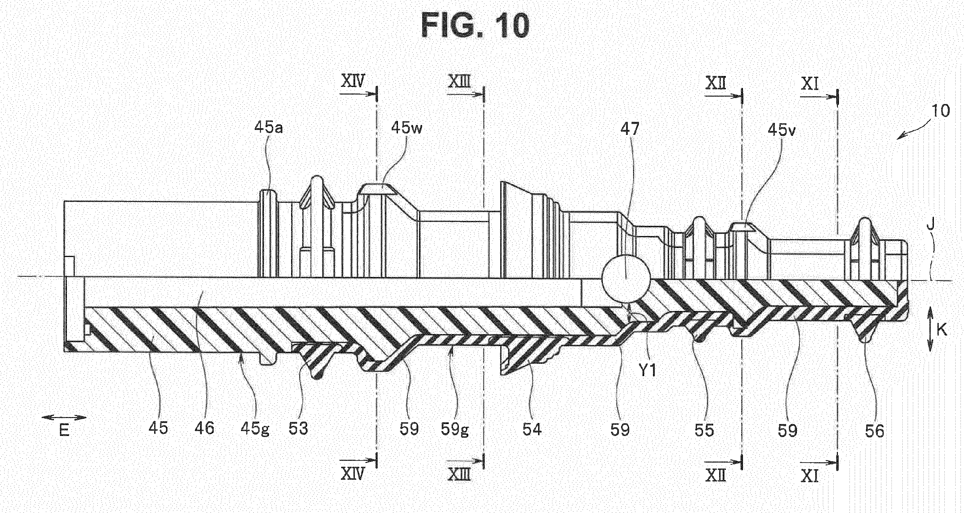

Fig. 8 is a top view of the conduit switching piston in Fig. 6 as seen from a VIII direction in Fig. 6, Fig. 9 is a side view of the conduit switching piston in Fig. 6 as seen from an IX direction in Fig. 6, and Fig. 10 is a view illustrating the conduit switching piston along an X-X line in Fig. 8, and which illustrates a cross-section of only one half of the conduit switching piston.

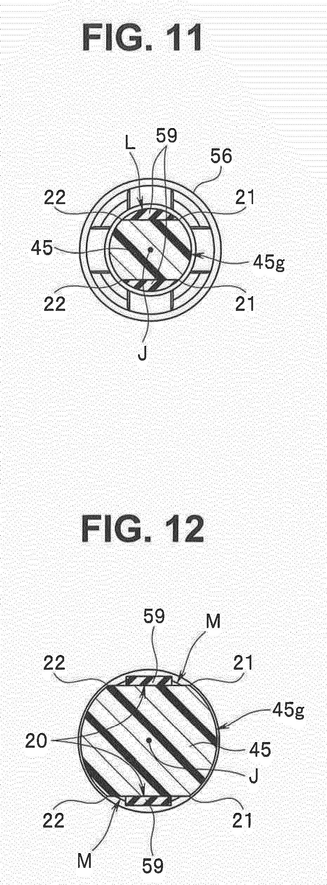

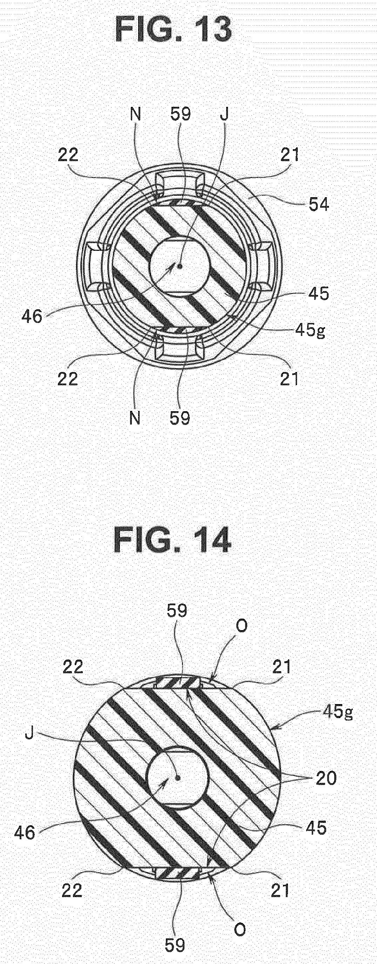

In addition, Fig. 11 is a cross-sectional view of the conduit switching piston along an XI-XI line in Fig. 10, Fig. 12 is a cross-sectional view of the conduit switching piston along an XII-XII line in Fig. 10, Fig. 13 is a cross-sectional view of the conduit switching piston along an XIII-XIII line in Fig. 10, and Fig. 14 is a cross-sectional view of the conduit switching piston along an XIV-XIV line in Fig. 10.

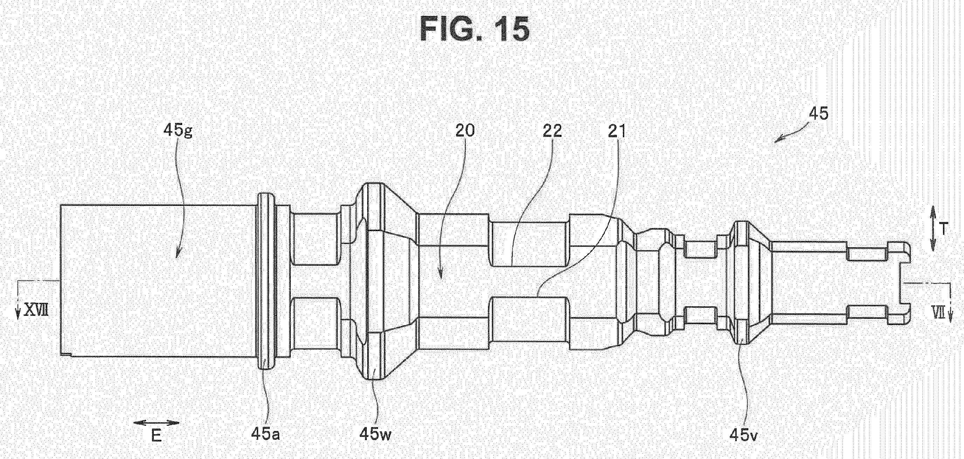

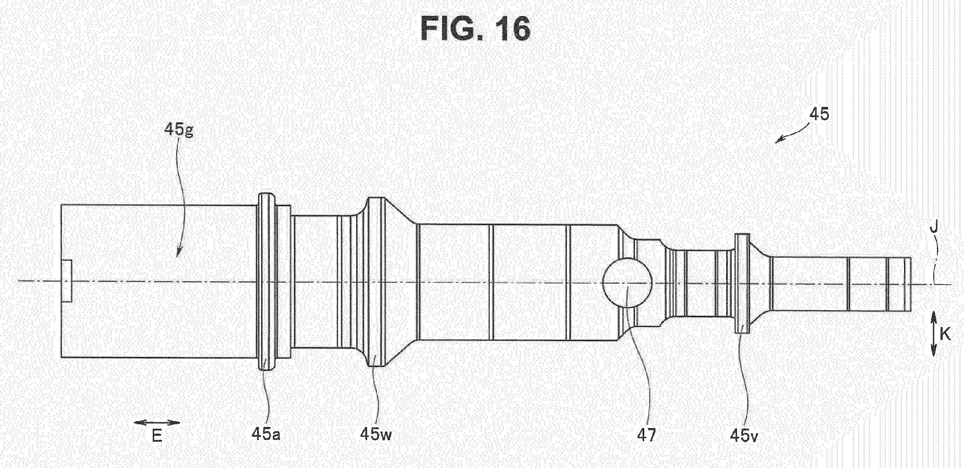

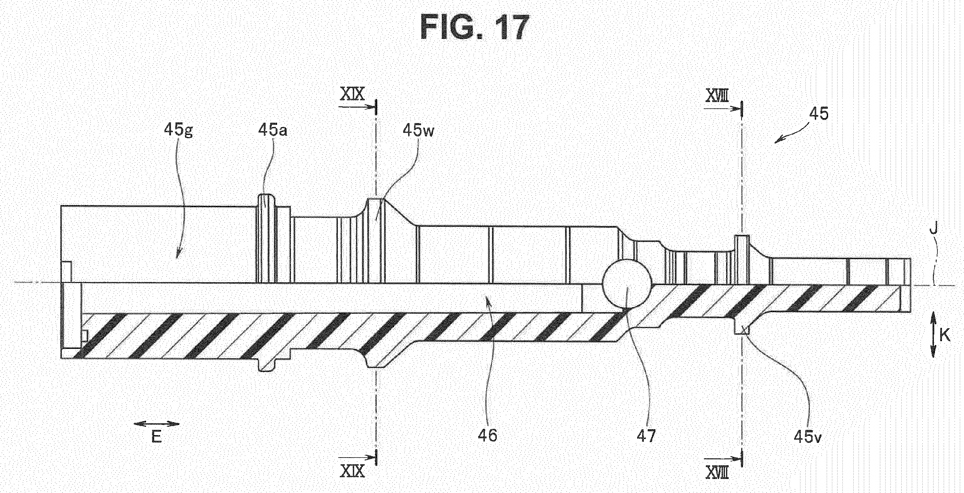

Fig. 15 is a top view of the piston main body in Fig. 7 as seen from an XV direction in Fig. 7, Fig. 16 is a side view of the piston main body in Fig. 7 as seen from an XVI direction in Fig. 7, and Fig. 17 is a view illustrating the piston main body along an XVII-XVII line in Fig. 15, and which illustrates a cross-section of only one half of the piston main body.

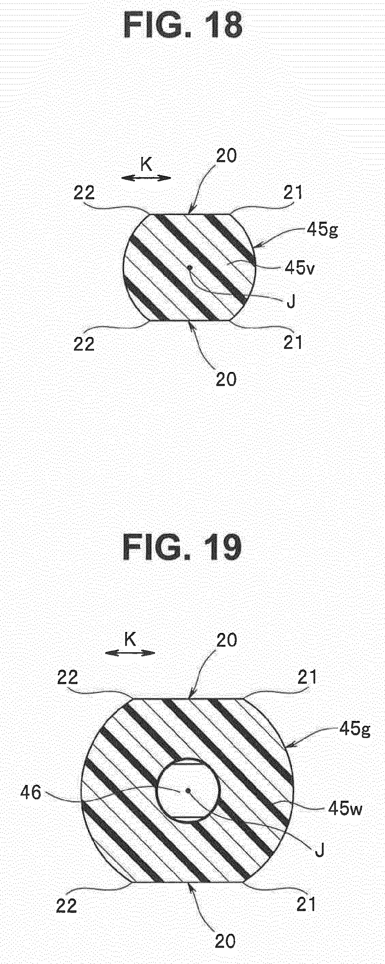

In addition, Fig. 18 is a cross-sectional view of the piston main body along an XVIII-XVIII line in Fig. 17, and Fig. 19 is a cross-sectional view of the piston main body along an XIX-XIX line in Fig. 17.

As illustrated in Fig. 7 and Fig. 15 to Fig. 17, a flat portion 20 is formed on the outer circumferential face 45g of the piston main body 45.

The flat portion 20 has two edge portions 21 and 22 that face each other on the outer circumferential face 45g and extend along the extending direction E, and is formed from the outward flange portion 45a to the lower end of the piston main body 45 along the extending direction E.

Specifically, as illustrated in Fig. 7 and Fig. 15 to Fig. 19, the flat portion 20 is formed with respect to the outer circumferential face 45g of the piston main body 45 by one part of the outer circumferential face 45g of the piston main body 45 whose cross-section in a radial direction K was formed in an approximately circular shape being cut out so that the flat portion 20 is parallel with a penetrating direction T of the through-hole 47.

Further, the flat portion 20 is formed at a position on the outer circumferential face 45g that is deviated by approximately 90° in the circumferential direction of the piston main body 45 from the through-hole 47.

Note that the flat portion 20 need not necessarily be formed at a position that is deviated by 90° in the circumferential direction from the through-hole 47. However, as described later, forming the flat portion 20 at a position that is deviated by 90° is preferable because, when the sealing unit 50 is injection molded using a mold on the outer circumferential face 45g of the piston main body 45, the piston main body 45 on which the sealing unit 50 is integrally formed can be easily taken out from the mold.

The flat portion 20 is also formed at the above described positions of the sliders 45w and 45v that butt against the inner circumferential face 35n of the cylinder 35, and needless to say the flat portion 20 is formed on the inner side in the radial direction K relative to the faces of the sliders 45w and 45v that butt against the inner circumferential face 35n. That is, regions of the sliders 45w and 45v at which the flat portion 20 is formed do not butt against the inner circumferential face 35n.

Further, as illustrated in Fig. 16 to Fig. 19, with respect to the outer circumferential face 45g of the piston main body 45, the flat portion 20 is formed on two sides with a central axis J along the extending direction E of the piston main body 45 being interposed between the two sides.

The sealing unit 50 is configured to have an elastic force by including the above described plurality of ring-shaped seal portions 53 to 56, and a connecting portion 59 that is formed integrally with the seal portions 53 to 56 and connects the seal portions 53 and 54, the seal portions 54 and 55, and the seal portions 55 and 56 that are respectively adjacent along the extending direction E.

Note that the sealing unit 50 is integrally formed on the outer circumferential face 45g of the piston main body 45 by injection molding a resin or the like into a mold with respect to the piston main body 45 that is formed from resin that was inserted into the mold.

Note that a material having self-adhesiveness with respect to the piston main body 45 is preferable as the material forming the sealing unit 50.

For example, in a case where the piston main body 45 is formed from polycarbonate, the sealing unit 50 is preferably formed from a polyester-based elastomer resin.

Further, in a case where the piston main body 45 is formed from polypropylene, the sealing unit 50 is preferably formed from a styrene-based thermoplastic elastomer resin.

In addition, in a case where the piston main body 45 is formed from a polysulfone, it has been found based on experimental results that the sealing unit 50 is preferably formed from silicon.

Consequently, since an adhesive need not be used for forming the sealing unit 50 on the outer circumferential face 45g of the piston main body 45, the molding cost and processing cost of the conduit switching piston 10 can be reduced.

Note that the material forming the sealing unit 50 is not limited to the above described materials, and may be any material that has self-adhesiveness with respect to the piston main body 45 and also has an elastic force.

As described above, the connecting portion 59 is a part that connects the plurality of seal portions 53 to 56 in the sealing unit 50, and as illustrated in Fig. 6 and Fig. 8 to Fig. 14, is formed with respect to the flat portion 20.

That is, by means of the flat portion 20, the connecting portion 59 is formed on the outer circumferential face 45g with a large contact area with respect to the outer circumferential face 45g, and hence the adhesiveness of the connecting portion 59 is enhanced.

Note that, as illustrated in Fig. 11 to Fig. 14, the connecting portion 59 is formed so as to be located within cut-out regions L, M, N and O formed with respect to the flat portion 20 by cutting out part of the outer circumferential face 45g of the piston main body 45.

Further, because the connecting portion 59 is formed in the flat portion 20, as illustrated in Fig. 9, the connecting portion 59 is formed so as to have a clearance between the inner circumferential face 35n of the cylinder 35 and the connecting portion 59.

Note that, as described above, on the outer circumferential face 45g of the piston main body 45, the sliders 45v and 45w are members configured to butt against the inner circumferential face 35n of the cylinder 35.

However, because the flat portion 20 is formed at the sliders 45v and 45w also, as illustrated in Fig. 9, the connecting portion 59 formed at the flat portion 20 of the sliders 45v and 45w is also formed at the flat portion 20 so as to have respective clearances P and Q with respect to the inner circumferential face 35n.

Thus, the connecting portion 59 is prevented from inhibiting the slidability of the sliders 45v and 45w with respect to the inner circumferential face 35n.

Furthermore, as illustrated in Fig. 13 and Fig. 14, the cross-sectional shape of the connecting portion 59 formed at the flat portion 20 of the sliders 45v and 45w preferably has a rectangular shape.

This is because, if the cross-sectional shape of the connecting portion 59 has a rectangular shape, as illustrated in Fig. 11 and Fig. 12, in comparison to a case where the cross-sectional shape is formed in a semicircular shape, places that have a thin edge cannot arise in the connecting portion 59 after being formed in the flat portion 20. It is therefore difficult for the connecting portion 59 to detach from the flat portion 20.

Note that, with regard to the cross-sectional shape of the connecting portion 59 to be formed in the flat portion 20 at regions other than the sliders 45v and 45w also, the connecting portion 59 may be formed to be a rectangular shape.

Further, as described above, on the outer circumferential face 45g of the piston main body 45, the flat portion 20 is formed at a position that is deviated in the circumferential direction relative to the through-hole 47.

Consequently, when the connecting portion 59 is injection molded with respect to the flat portion 20, the material forming the sealing unit 50 is prevented from leaking out from the through-hole 47, and furthermore the shape of the mold can be made a simple shape since there is no necessity to form the mold in a shape that avoids the through-hole 47.

For these reasons, the occurrence of molding defects or burrs in the sealing unit 50 can be prevented, and the mold can be manufactured at a low cost.

In addition, because the flat portion 20 is formed on two sides of the outer circumferential face 45g of the piston main body 45 with the central axis J being interposed between the two sides, the connecting portion 59 is also formed on both sides with the central axis J being interposed between the two sides.

That is, since the material forming the connecting portion 59 can be filled into the mold from both sides, the filling property is enhanced, and in addition, because it is difficult for molding defects to occur in the connecting portion 59, the manufacturing yield of the conduit switching piston 10 increases.

Note that, if the aforementioned advantage is to be disregarded, the connecting portion 59 and the flat portion 20 may be formed at one place on only one side with respect to the outer circumferential face 45g of the piston main body 45 as in the conventional configuration.

The remaining configuration of the conduit switching apparatus 13 is the same as the configuration of the conventional conduit switching apparatus 13.

Thus, in the present embodiment a configuration has been described in which, in the conduit switching piston 10, the flat portion 20 is formed along the extending direction E on the outer circumferential face 45g of the piston main body 45.

Further, it has been described that after the sealing unit 50 is injection molded onto the outer circumferential face 45g, the connecting portion 59 configured to connect the plurality of ring-shaped seal portions 53 to 56 along the extending direction E is formed on the flat portion 20.

Consequently, it is not necessary to form a groove portion for forming the sealing unit 50 in the outer circumferential face 45g as in the conventional configuration.

Therefore, the shape of a mold to be used when forming the sealing unit 50 is simple and the sealing unit 50 can be formed inexpensively, and furthermore a large contact area of the connecting portion 59 with respect to the flat portion 20 can be secured. Consequently, the sealing unit 50 can be injection molded so that the sealing unit 50 is firmly bonded to the outer circumferential face 45g.

Further, because the flat portion 20 is formed at a position on the outer circumferential face 45g that is deviated in the circumferential direction of the piston main body 45 from the through-hole 47, the shape of a mold for forming the sealing unit 50 can be simplified and the mold can be made at a low cost.

In addition, it has been described that the connecting portion 59 is formed on the flat portion 20 so as to have a clearance with respect to the inner circumferential face 35n of the cylinder 35, and that a cross-section of the connecting portion that is formed on the flat portion 20 of the sliders 45v and 45w is formed in a rectangular shape.

Therefore, because the connecting portion 59 does not contact the inner circumferential face 35n, the sliding resistance of the piston main body 45 is not increased by the connecting portion 59.

As described in the foregoing, the conduit switching piston 10 and the endoscope 102 can be provided in which the conduit switching piston 10 has a configuration in which the plurality of seal portions 53 to 56 are integrally formed with respect to the outer circumferential face 45g of the piston main body 45 inexpensively and with an enhanced fixing strength.

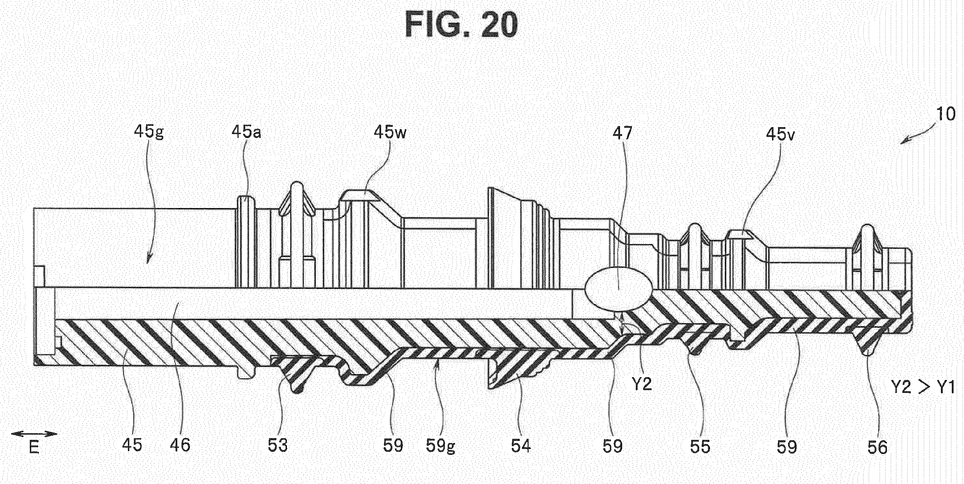

A modification will now be described using Fig. 20. Fig. 20 is a view illustrating a modification of the conduit switching piston in which the shape of a through-hole formed in the piston main body in Fig. 10 is made elliptical, and which illustrates a cross-section of only one half of the conduit switching piston.

As illustrated in Fig. 20, the shape of the through-hole 47 may be made an elliptical shape that has the same area as a circle and in which the long axis is along the extending direction E.

According to this configuration, while keeping the same amount with respect to the air feeding amount for which the through-hole 47 is used, as illustrated in Fig. 10 and Fig. 20, it is possible to secure a wall thickness Y2 in the radial direction K of a region in which the through-hole 47 is formed in the piston main body 45 that is greater than a wall thickness Y1 in the case of the circular through-hole 47 (Y2 > Y1). Thus, the strength of the piston main body 45 can be further improved.

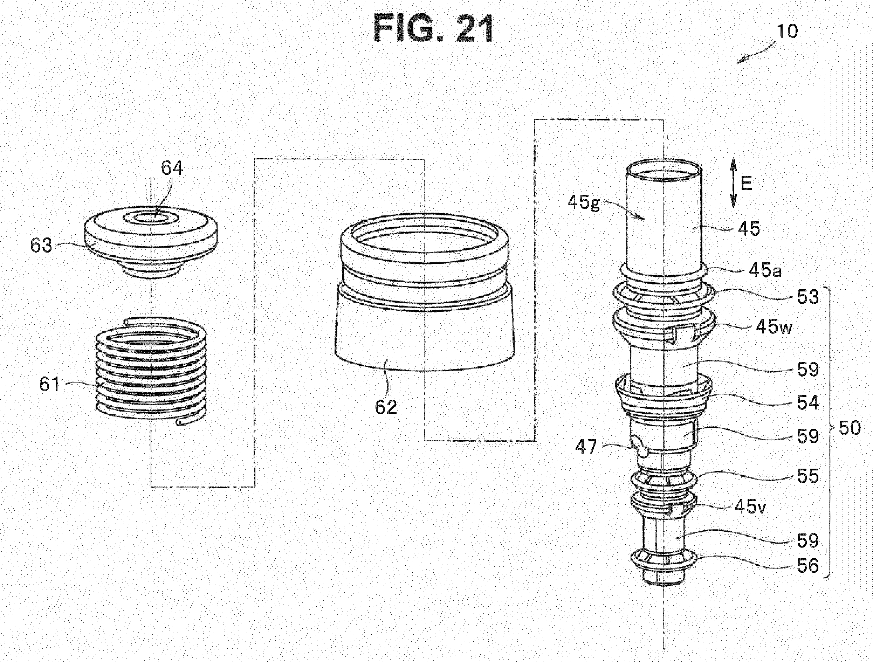

Another modification will be described hereunder using Fig. 21. Fig. 21 is an exploded perspective view of a piston main body on which the sealing unit of the conduit switching piston shown in Fig. 3 is formed, an enclosing member, an urging spring and an air/water feeding button.

In the present embodiment described above, it is described that the air/water feeding button 63 is fixed by being screwingly attached to the outer circumference of the top end of the piston main body 45.

Regardless of the above description, as illustrated in Fig. 21, after the piston main body 45 on which the sealing unit 50 is formed, the enclosing member 62, the urging spring 61 and the air/water feeding button 63 are assembled, the air/water feeding button 63 may be fixed by ultrasound welding to the top end of the piston main body 45.

According to this configuration, not only is it not necessary to use an adhesive for fixing the air/water feeding button 63, the number of components can also be reduced relative to a structure in which the air/water feeding button 63 is fixed via an unshown plurality of components to the top end of the piston main body 45, and hence the manufacturing cost can be reduced.

Note that, in the foregoing present embodiment, it is described that the conduit switching piston 10 switches communication states of air feeding conduits and water feeding conduits.

Regardless of the above description, it is needless to say that the conduit switching piston 10 is also applicable to a configuration that switches communication states of other conduits, and is also applicable to a configuration that switches communication states of three or more conduits.

The present application claims priority from Japanese Patent Application No. 2016-033400 filed in Japan on February 24, 2016, the contents of which are hereby incorporated by reference in their entirety into the description, claims and drawings of the present application.

## Claims

1. A conduit switching piston that is fitted and inserted in an advanceable and retractable manner into a cylinder to which a plurality of conduits are connected, the conduit switching piston being configured to switch communication states of the plurality of conduits, comprising:
a shaft member;
a flat portion formed along an extending direction of the shaft member on an outer circumferential face of the shaft member, and having two edge portions facing each other on the outer circumferential face and extending along the extending direction;
a plurality of seal portions formed having a set interval with respect to each other along the extending direction on the outer circumferential face of the shaft member, and configured to elastically contact an inner circumferential face of the cylinder; and
a connecting portion formed on the flat portion integrally with the seal portions, and configured to connect the seal portions which are adjacent along the extending direction.

2. The conduit switching piston according to claim 1, wherein:
a cross-section of the shaft member in a radial direction is formed in a circular shape, and the flat portion is formed by cutting out one part of the outer circumferential face of the shaft member; and
the connecting portion is located within a cut-out region of the shaft member.

3. The conduit switching piston according to claim 2, wherein a cross-sectional shape in the radial direction of the connecting portion has a rectangular shape.

4. The conduit switching piston according to claim 1, wherein the flat portion is formed on an inner side in a radial direction of the shaft member relative to a face that butts against the inner circumferential face of the cylinder of the shaft member.

5. The conduit switching piston according to claim 1, wherein a clearance is formed between an outer circumferential face of the connecting portion and the inner circumferential face of the cylinder.

6. The conduit switching piston according to claim 1, wherein, on the outer circumferential face of the shaft member, the flat portion is formed on each of two sides opposite to each other with a central axis of the shaft member being interposed between the two sides.

7. The conduit switching piston according to claim 1, wherein:
a through-hole which penetrates in a radial direction of the shaft member is formed in the shaft member; and
the flat portion is formed so as to be parallel to a penetrating direction of the through-hole.

8. An endoscope in which a conduit switching piston according to claim 1 is mounted.

9. The endoscope according to claim 8, wherein the conduit switching piston is freely fitted and inserted in the cylinder that is provided in an operation portion to be grasped by an operator.
